# EUROPEAN PATENT SPECIFICATION

(11) **EP 3 438 363 B1**
(45) Date of publication and mention of the grant of the patent: **24.06.2020**
(21) Application number: 18188949.4
(22) Date of filing: 09.02.2015
(51) Int. Cl.: B05C 3/08, E04B 1/62, A61F 13/00, B32B 27/12, B32B 29/02, B32B 27/28, B32B 17/00, B32B 7/14, B32B 7/06, B32B 5/26, B32B 5/02, B05D 1/28, B05D 7/00, B05B 13/04, B05C 9/06, B05C 5/02, B32B 27/36, B32B 27/32, B32B 27/06, B32B 7/12, B05B 13/02, B05C 3/10, B05C 7/04, B05C 9/08

(54) **METHOD FOR FORMING ARTICLES WITH NON-UNIFORM COATINGS**
VERFAHREN ZUR HERSTELLUNG VON ARTIKELN MIT UNGLEICHMÄSSIGEN BESCHICHTUNGEN
PROCÉDÉ POUR LA FORMATION D'ARTICLES PORTANT DES REVÊTEMENTS NON UNIFORMES

(30) Priority: 18.02.2014 US 201461941160 P
(43) Date of publication of application: 06.02.2019
(62) Divisional of application: 15751593.3
(73) Proprietor: 3M Innovative Properties Company, St. Paul, MN 55133-3427 (US)
(72) Inventor: Maier, Gary W., St. Paul, MN Minnesota 55133-3427 (US); Fronek, Daniel R., St. Paul, MN Minnesota 55133-3427 (US); Hollo, Alan J., St. Paul, MN Minnesota 55133-3427 (US); Widenbrant, Martin J. O., St. Paul, MN Minnesota 55133-3427 (US); Meredyk, Wayne D., St. Paul, MN Minnesota 55133-3427 (US)
(74) Representative: Gabriel, Kiroubagaranne

(56) References cited:
- WO-A1-2013/168777
- US-A- 3 831 342
- US-A1- 2005 028 938
- US-B1- 6 176 961

## Description

### TECHNICAL FIELD

The present disclosure relates generally to the application of coatings to substrates, and more particularly to the application of non-uniform coatings.

### BACKGROUND

The fabrication of numerous commercial products includes the step of applying a coating to a substrate in the form of a sheet or a web of indefinite length. For some applications it is desirable to have an overall uniform coating on the substrate, while in others it is desirable to apply a non-uniform coating in the form of, e.g., a multiplicity of stripes. The non-uniform coating may be applied directly to the substrate or to an intermediate surface with subsequent transfer to the substrate, or may be applied superimposed over an earlier uniform coating on the substrate. For example, the use of needle tubes to apply stripes of a coating material to a coating roll has been described, for example in Maier, PCT Patent Publication WO2011/087657 Patent Application Publication US 2005/0028938 describes systems and methods for depositing glue mixtures in defined patterns on workpieces transported on a conveyor. Documents WO 2013/168777; US 6,176,961 and US 3,831,342 disclose coating, bonding and adhesive applying devices and methods.

### SUMMARY

The present invention describes a method for applying a coating material to a substrate as in claim 1. In one aspect, the present disclosure describes a method of applying a coating material onto a substrate, including providing a distribution manifold having a cavity and a plurality of dispensing outlets in fluid communication with the cavity; creating relative motion between the substrate and the dispensing outlets in a first direction; and dispensing a coating material from the dispensing outlets while simultaneously translating the plurality of dispensing outlets in a second direction non-parallel to the first direction.

In a second aspect, the present disclosure describes a coated article, including a substrate having a major surface extending in a longitudinal direction between a first longitudinal edge and a second longitudinal edge, and a cross direction between the edges; a first coating that is continuous in the longitudinal direction and discontinuous in the cross direction, disposed on the major surface in a first pattern that varies relative to the longitudinal edges; and a second coating that is continuous in the longitudinal direction and discontinuous in the cross direction, disposed on the substrate in a second pattern.

In some exemplary embodiments of the method, the plurality of dispensing outlets forms an array. In these or other embodiments, the plurality of dispensing outlets is comprised of a plurality of needle tubes in fluid communication with the cavity. In some of the embodiments where needle tubes are present, an alignment bar for securing a spacing between the needle tubes is employed. In some of these embodiments, the spacing is substantially uniform, while in other embodiments the spacing is substantially non-uniform.

In some embodiments, it is the distribution manifold that is translated in the second direction, thereby translating the plurality of dispensing outlets in the second direction. In other alternate embodiments where needle tubes are employed, the distribution manifold is stationary and the needle tubes are translated in the second direction, translating the plurality of dispensing outlets in the second direction. In these latter embodiments, translating the needle tubes is conveniently accomplished by translating an alignment bar.

In some convenient embodiments, the second direction is perpendicular to the first direction. In particular when the substrate is in the form of web of indefinite length, the first direction will be in the direction of indefinite length, and the second direction will the perpendicular to the first direction. Persons of skill in the art might describe these directions as the "machine direction" and the "cross direction," respectively, relative to the web of indefinite length. In these or other embodiments, the translating may be an oscillatory fashion.

In some embodiments, the method will further include providing a second distribution manifold, also having a cavity and a plurality of second dispensing outlets in fluid communication with the cavity; and dispensing a second coating material from the second dispensing outlets. In some of these embodiments, the second dispensing outlets are simultaneously translated in a direction non-parallel to the first direction. In others of these embodiments, the second dispensing outlets are maintained still relative to the substrate. In the embodiments where the second dispensing outlets are simultaneously translated in a direction non-parallel to the first direction, the manner and/or frequency of the translation may be similar to, or distinct from, the translation undergone by the first dispensing outlets.

In embodiments where a second distribution manifold is employed, the first and second coating materials may be the same or different. In either of these types of embodiments, the regions of the substrate coated by the first and second coating materials may overlap, or they may be completely discrete. In some of the embodiments of the former type, coating-free zones completely surrounded by the first and the second coating materials are formed on the substrate.

In any of the above described embodiments, the distribution manifold separates into a manifold containing the cavity, and a removable cartridge having the dispensing outlets. In some of these, the dispensing outlets will comprise needle tubes in fluid communication with the removable cartridge.

### Listing of Exemplary Embodiments

A. A method of applying a coating material to a substrate, comprising:
   providing a distribution manifold having a cavity and a plurality of dispensing outlets in fluid communication with the cavity; creating relative motion between a substrate and the dispensing outlets in a first direction; and dispensing coating material from the dispensing outlets while simultaneously translating the plurality of dispensing outlets in a second direction non-parallel to the first direction.
B. The method according to embodiment A wherein the distribution manifold is translated in the second direction, thereby translating the plurality of dispensing outlets in the second direction, optionally wherein the substrate is moved in the first direction relative to the distribution manifold.
C. The method according to embodiment A or B wherein the plurality of dispensing outlets forms an array of dispensing outlets.
D. The method according to any one of embodiment A, B or C wherein the plurality of dispensing outlets is comprised of a plurality of needle tubes in fluid communication with the cavity.
E. The method according to embodiment D wherein the distribution manifold further comprises an alignment bar for securing a relative spacing between each needle tube, and further wherein the relative spacing is substantially uniform.
F. The method according to embodiment D wherein the distribution manifold further comprises an alignment bar for securing a relative spacing between each needle tube, and further wherein the relative spacing is substantially non-uniform.
G. The method according to any one of the preceding embodiments wherein the distribution manifold may be separated into a manifold chamber containing the cavity, and a removable cartridge having the plurality of needle tubes.
H. The method according to any one of the preceding embodiments wherein the second direction is perpendicular to the first direction.
I. The method according to any one of the preceding embodiments wherein translating is in anoscillatory fashion.
J. The method according to any one of the preceding embodiments further comprising providing a second distribution manifold having a cavity and a plurality of second dispensing outlets in fluid communication with the cavity, and dispensing a second coating material from the second dispensing outlets.
K. The method according embodiment J wherein the second distribution manifold is simultaneously translated in a direction non-parallel to the first direction.
L. The method according to embodiments J or K wherein coating-free zones completely surrounded by the first and the second coating materials are formed on the substrate.
M. The method according to embodiment L wherein the first and second coating materials are different.
N. The method according to embodiments E or F wherein the translating is accomplished by moving the alignment bar.
O. A coated article, comprising:
   a substrate having a major surface extending in a longitudinal direction between a first longitudinal edge and a second longitudinal edge, and in a cross direction between the edges;
   a first coating that is continuous in the longitudinal direction and discontinuous in the cross direction, disposed on the major surface in a first pattern that varies relative to the longitudinal edges; and
   a second coating that is continuous in the longitudinal direction and discontinuous in the cross direction, disposed on the substrate in a second pattern.
P. The article according to embodimens O wherein the second pattern is parallel to the longitudinal edges.
Q. The article according to embodiment P wherein the second pattern does not contact the first
   pattern.
R. The article according to embodiment P wherein the second pattern contacts the first pattern.
S. The article according to claim P wherein the second coating pattern varies relative to the longitudinal edges.
T. The article according to embodiment S wherein the second pattern does not contact the first pattern.
U. The article according to embodiment S wherein the second pattern contacts the first pattern, and wherein a plurality of coating-free zones is formed on the substrate wherein each zone is completely surrounded by both the first and the second coatings.
V. The article according to any one of embodiments O-U wherein the first and second coating materials are different.
W. The article according to embodiment V wherein the first and second coating materials are adhesives.
X. The article according to embodiment W wherein the first and second coating materials are formulated so as to particularly adhere advantageously to two distinct surface conditions.
Y. A self-adhesive, vapor permeable air and moisture barrier membrane comprising:
   a substrate having opposed a first major surface and a second opposed major surface extending in a longitudinal direction between a first longitudinal edge and a second longitudinal edge, and in a cross direction between the edges;
   a first layer of a first adhesive material that is continuous in the longitudinal direction and discontinuous in the cross direction, disposed on the major surface in a first pattern that varies relative to the longitudinal edges; and
   a second layer of a second adhesive material that is continuous in the longitudinal direction and discontinuous in the cross direction, disposed on the substrate in a second pattern.
Z. The membrane of embodiment Y, wherein the substrate comprises a (co)polymer selected from polyethylene terephthalate, polylactic acid, polyethylene napthalate, polyimide, polycarbonate, polyethylene, polypropyelene, polybutylene, cyclo olefins, and combinations thereof.
AA. The membrane of embodiments Y or Z, wherein the first and second adhesive materials are different adhesive materials, optionally wherein the first and second adhesive materials each comprises a pressure sensitive adhesive selected from a solvent-based pressure sensitive adhesive, a solvent-free hot melt pressure sensitive adhesive, a water-based pressure sensitive adhesive, or a combination thereof.
BB. The membrane of any one of embodiments Y, Z or AA wherein the at least one non-uniform adhesive layer forms a pattern selected from circles with adhesive free centers, polygons with adhesive free centers, or a plurality of adhesive strips with intervening adhesive free strips.
CC. A self-adhesive, vapor permeable air and moisture barrier membrane according to the method of any one of embodiments A-N.
DD. A membrane according to any one of embodiments Y, Z, AA, BB, or CC, wherein the membrane is applied to an architectural structure.

Various aspects and advantages of exemplary embodiments of the disclosure have been summarized. The above Summary is not intended to describe each illustrated embodiment or every implementation of the present certain exemplary embodiments of the present disclosure. The Drawings and the Detailed Description that follow more particularly exemplify certain preferred embodiments using the principles disclosed herein.

### Brief Description of the Drawings

FIG. 1 is a perspective view of a coating apparatus suitable for carrying out the method of the present disclosure.
FIG. 2 is a perspective view of a dual coating apparatus.
FIG. 3 is a perspective view of an alternative coating apparatus.
FIG. 4 is a plan view of length of coated substrate prepared by the dual coating apparatus of FIG. 2.
FIG. 5 is a plan view of different length of coated substrate prepared by the dual coating apparatus of FIG. 2.

In the drawings, like reference numerals indicate like elements. While the above-identified drawing, which may not be drawn to scale, sets forth various embodiments of the present disclosure, other embodiments are also contemplated, as noted in the Detailed Description. In all cases, this disclosure describes the presently disclosed disclosure by way of representation of exemplary embodiments and not by express limitations. It should be understood that numerous other modifications and embodiments can be devised by those skilled in the art, which fall within the scope and spirit of this disclosure.

### DETAILED DESCRIPTION

Recently, the use of needle tubes to apply stripes of coating material to a coating roll was disclosed in co-pending and co-assigned PCT Patent Publication WO2011/087657. Other coating apparatus making use of needle tubes were disclosed in co-pending and co-assigned PCT Patent Publication WO2011/159276 and PCT Patent Publication WO2013/090575.

The present disclosure describes coating methods including dispensing coating material from a plurality of dispensing outlets while simultaneously translating the plurality of dispensing outlets. In convenient embodiments, the plurality of dispensing outlets is at the distal ends of needle tubes in fluid communication with the cavity of a distribution manifold.

For the following Glossary of defined terms, these definitions shall be applied for the entire application, unless a different definition is provided in the claims or elsewhere in the specification.

### Glossary

Certain terms are used throughout the description and the claims that, while for the most part are well known, may require some explanation. It should understood that, as used herein:
The term "homogeneous" means exhibiting only a single phase of matter when observed at a macroscopic scale.
The terms "(co)polymer" or "(co)polymers" includes homopolymers and copolymers, as well as homopolymers or copolymers that may be formed in a miscible blend, *e.g.,* by coextrusion or by reaction, including, *e.g*., transesterification. The term "copolymer" includes random, block and star (e.g. dendritic) copolymers.
The term "(meth)acrylate" with respect to a monomer, oligomer or means a vinyl-functional alkyl ester formed as the reaction product of an alcohol with an acrylic or a methacrylic acid.
The term "adjoining" with reference to a particular layer means joined with or attached to another layer, in a position wherein the two layers are either next to (i.e., adjacent to) and directly contacting each other, or contiguous with each other but not in direct contact (i.e., there are one or more additional layers intervening between the layers).
By using terms of orientation such as "atop", "on", "over," "covering", "uppermost", "underlying" and the like for the location of various elements in the disclosed coated articles, we refer to the relative position of an element with respect to a horizontally-disposed, upwardly-facing substrate. However, unless otherwise indicated, it is not intended that the substrate or articles should have any particular orientation in space during or after manufacture.
By using the term "overcoated" to describe the position of a layer with respect to a substrate or other element of an article of the present disclosure, we refer to the layer as being atop the substrate or other element, but not necessarily contiguous to either the substrate or the other element.
By using the term "separated by" to describe the position of a layer with respect to other layers, we refer to the layer as being positioned between two other layers but not necessarily contiguous to or adjacent to either layer.
The terms "about" or "approximately" with reference to a numerical value or a shape means +/- five percent of the numerical value or property or characteristic, but expressly includes the exact numerical value. For example, a viscosity of "about" 1 Pa-sec refers to a viscosity from 0.95 to 1.05 Pa-sec, but also expressly includes a viscosity of exactly 1 Pa-sec. Similarly, a perimeter that is "substantially square" is intended to describe a geometric shape having four lateral edges in which each lateral edge has a length which is from 95% to 105% of the length of any other lateral edge, but which also includes a geometric shape in which each lateral edge has exactly the same length.
The term "substantially" with reference to a property or characteristic means that the property or characteristic is exhibited to a greater extent than the opposite of that property or characteristic is exhibited. For example, a substrate that is "substantially" transparent refers to a substrate that transmits more radiation (e.g. visible light) than it fails to transmit (e.g. absorbs and reflects). Thus, a substrate that transmits more than 50% of the visible light incident upon its surface is substantially transparent, but a substrate that transmits 50% or less of the visible light incident upon its surface is not substantially transparent.
As used in this specification and the appended embodiments, the singular forms "a", "an", and "the" include plural referents unless the content clearly dictates otherwise. Thus, for example, reference to fine fibers containing "a compound" includes a mixture of two or more compounds. As used in this specification and the appended embodiments, the term "or" is generally employed in its sense including "and/or" unless the content clearly dictates otherwise.
   As used in this specification, the recitation of numerical ranges by endpoints includes all numbers subsumed within that range (e.g. 1 to 5 includes 1, 1.5, 2, 2.75, 3, 3.8, 4, and 5).
Unless otherwise indicated, all numbers expressing quantities or ingredients, measurement of properties and so forth used in the specification and embodiments are to be understood as being modified in all instances by the term "about." Accordingly, unless indicated to the contrary, the numerical parameters set forth in the foregoing specification and attached listing of embodiments can vary depending upon the desired properties sought to be obtained by those skilled in the art utilizing the teachings of the present disclosure. At the very least, and not as an attempt to limit the application of the doctrine of equivalents to the scope of the claimed embodiments, each numerical parameter should at least be construed in light of the number of reported significant digits and by applying ordinary rounding techniques.

Exemplary embodiments of the present disclosure may take on various modifications and alterations without departing from the scope of the present disclosure. Accordingly, it is to be understood that the embodiments of the present disclosure are not to be limited to the following described exemplary embodiments, but is to be controlled by the limitations set forth in the claims.

### Exemplary Coating Apparatus and Processes

Various exemplary embodiments of the disclosure will now be described with particular reference to the Drawings.

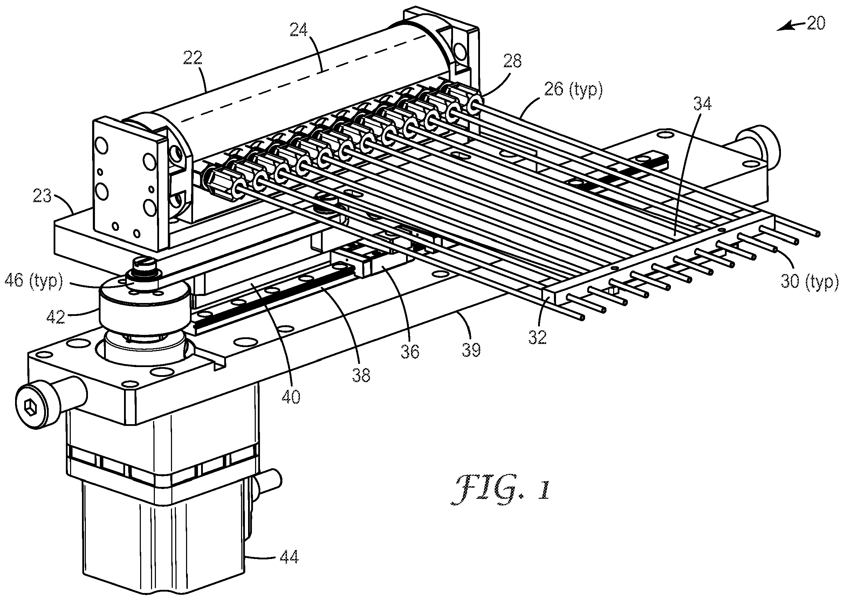

Referring to FIG. 1, a perspective view of a coating apparatus 20 suitable for carrying out the methods of the present disclosure is illustrated. The coating apparatus 20 includes a distribution manifold 22 on a support 23. Distribution manifold 22 has a cavity 24 internally (rendered in dotted lines in this Figure). A plurality of needle tubes 26 is in fluid communication with cavity 24. Quick release fittings 28 are provided for convenience in cleaning the apparatus 20 between uses, and also to conveniently change the width of the coated pattern to be generated by the apparatus. Quick release fittings from Swagelok of Solon, OH are considered suitable. Coating material is supplied to the cavity 24 via an inlet port (on the far side in this view) from a pump.

Needle tubes 26 end in a plurality of dispensing outlets 30, by extension also in fluid communication with the cavity 24. In some embodiments such as the one illustrated, the dispensing outlets 30 form an array. The array may be linear as illustrated in this Figure, but nonlinear arrays may be convenient for some purposes. In some convenient embodiments, the dispensing outlets 30 are evenly spaced along the distribution manifold 22, but non-uniform spacing may also be convenient, e.g. when the article coated by apparatus 20 is to be slit in a downstream operation into several portions.

In the illustrated embodiment, the spacing between the needle tubes 26, and by extension the dispensing outlets 30, is secured by an alignment bar 32. Alignment bar 32 is conveniently attached to a plate 34 which is in turn attached to a slide 36. Slide 36 is slideably mounted on a track 38 attached to a frame 39. The motion of slide 36 along track 38 is controlled by a bar 40 pivotally mounted on slide 36. The other end of bar 40 is pivotally mounted on rotor 42, which can be rotated by motor 44. Rotor 42 has several attachment holes 46 at diverse distances from the axis of rotation of motor 44. Though this mechanism, the slide 36 can be placed in reciprocating motion by activating motor 44. By the choice of which attachment hole 46 is selected for the attachment of bar 40, the amplitude of the reciprocating motion is easily changed. The frequency of the reciprocating motion is easily controlled by the speed setting selected for motor 44.

In some embodiments, the needle tubes are conveniently made from stainless steel. Other materials that can be formed into hollow conduits, such as polymers, can also be used. Further, in embodiments such as the one illustrated in FIG. 1 which includes alignment bar 32 and rigid plate 34, it is possible to use non-rigid materials such as silicone rubber tubing to form needle tubes 26.

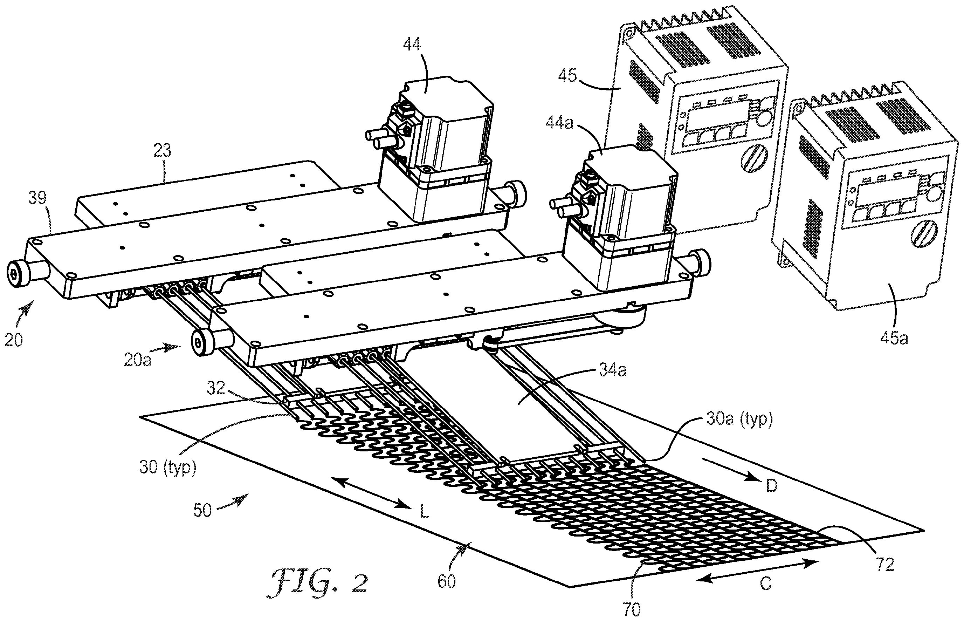

Referring now to FIG. 2, a dual coating apparatus 50 is illustrated. Dual coating apparatus 50 includes a first distribution manifold 20 and a second distribution manifold 20a. Conveniently, distribution manifold 20 and a second distribution manifold 20a are both constructed as described in FIG. 1, although there is no necessity when there are two or more distribution manifolds for them to be similar. In this Figure, motor controllers 45 and 45a, which control and power motors 44 and 44a on first distribution manifold 20 and second distribution manifold 20a, respectively, are shown. First distribution manifold 20 and a second distribution manifold 20a have first and second dispensing outlets 30 and 30a respectively, positioned adjacent to a substrate 60.

The substrate 60 has a longitudinal direction "L" and a cross direction "C". In this Figure, substrate 60 is being conveyed past dispensing outlets 30 and 30a in a first direction "D". No specific means for conveying the substrate 60 is critical to the utility of the present disclosure, and in general any of the diverse mechanisms known to artisans for this purpose will suffice. While substrate 60 is being conveyed, the first plurality of dispensing outlets 30 is simultaneously translated in a second direction that is non-parallel to the first direction. This is accomplished by operating motor 44 to move alignment bar 32. In the depicted embodiment, that second direction conveniently happens to be identical to cross direction "C," but this identity is not critical to the utility of the present disclosure.

The combination of the movement of substrate 60 in direction "D" while first plurality of dispensing outlets 30 is reciprocated in the "C" direction causes first coating material being dispensed from first plurality of dispensing outlets 30 to be laid onto substrate 60 in sinusoidal patterns 70. Reciprocation rates of between about 0.16 Hz to 6.16 Hz have been found to be convenient. In this Figure, second plurality of dispensing outlets 30a is not being reciprocated, which causes second coating material being dispensed from second plurality of dispensing outlets 30a to be laid onto substrate 60 in straight patterns 72.

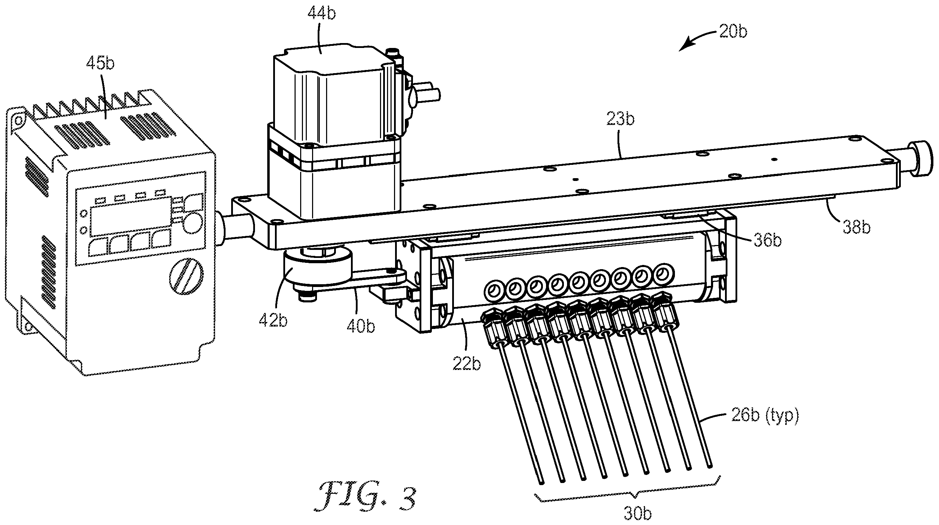

Referring now to FIG. 3, a perspective view of an alternative coating apparatus 20b is illustrated. Coating apparatus 20b is similar in some ways to coating apparatus 20 of FIG. 1, except that the entire distribution manifold 22b is mounted onto slide 36b, which is itself slidably mounted to track 38b on support 23b. When motor controller 45b operates motor 44b, turning rotor 42b, bar 40b reciprocates distribution manifold 22b directly. This in turn reciprocates needle tubes 26b and thereby reciprocates dispensing outlets 30b. This variant may be convenient when the total displacement in the second direction is low, or the speed of conveying the substrate is slower.

A rotor and bar mechanism as depicted in FIGS 1-3 is not the only mechanism contemplated for translating the dispensing outlets. For example, a stepper motor could be connected by a mechanism to either the distribution manifold or to an alignment bar. A linear displacement transducer could be employed similarly. Such alternatives could be synchronized to the conveying speed of the substrate so that complex non-sinusoidal patterns could be laid down for the first and/or second coating material.

### Exemplary Coated Articles

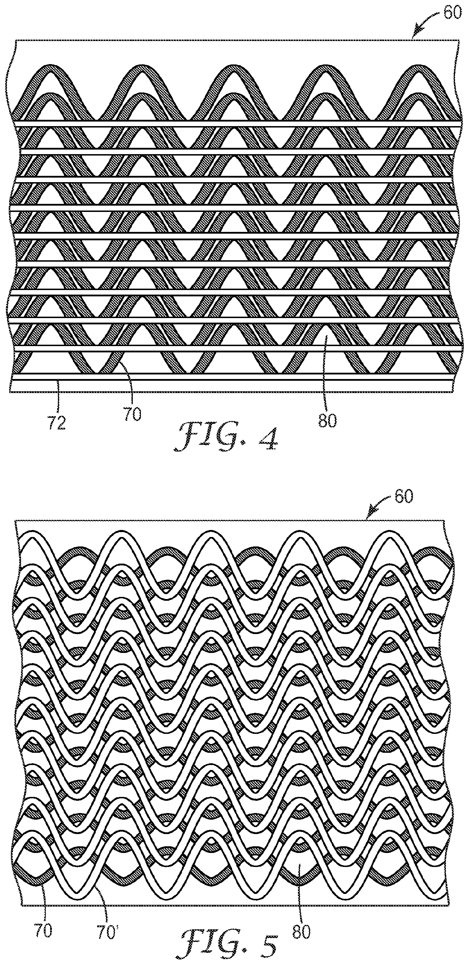

Referring now to Referring now to FIG. 4, a plan view of length of coated substrate 60 prepared by the dual coating apparatus of FIG. 2, is illustrated. On substrate 60, sinusoidal patterns 70 laid down in a first coating material overlap straight patterns 72 laid down in a second coating material. Such an overlap is not a requirement of the disclosure even when first and second distribution manifolds are in use; the positioning and spacing of the first and second distribution outlets can be arranged so that there is no overlap. The first and the second coating materials may be same or different. In some applications, e.g. wound care products, it may be convenient to create coating-free zones completely surrounded by both the first and the second coating materials on the substrate. Zone 80 is one such zone. The first and the second coating materials may independently be an adhesive. In some applications, the first and second coating materials are both adhesives, formulated so as to particularly adhere advantageously to two distinct surface conditions. For example, in a wound dressing, it may be desirable lay down one pattern with an adhesive well adapted to adhere to dry skin, while additionally laying down one pattern with an adhesive well adapted to adhere to moist skin. The product will perform regardless of the presenting condition of the patient.

Referring now to FIG. 5, a plan view of different length of coated substrate 60 prepared by the dual coating apparatus of FIG. 2, is illustrated. On substrate 60, first sinusoidal patterns 70 laid down in a first coating material overlap second sinusoidal patterns 70' laid down in a second coating material. As in the embodiment of FIG. 4, such an overlap is not a requirement of the disclosure, and the first and the second coating materials may be same or different. In some applications, e.g. wound care products, it may be convenient to create coating-free zones completely surrounded by both the first and the second coating materials on the substrate. Zone 80 is one such zone.

### Self-adhering, Vapor Permeable Air Barrier Membranes

In some presently preferred embodiments, the coated article is a self-adhering, vapor permeable air barrier membrane. The vapor permeable membranes of the present disclosure are generally flexible sheets or films, normally supplied in roll form, which are permeable to the passage of water in vapor form. The sheet or film may be microporous, microperforated or some other type of vapor permeable sheet or film. A microporous sheet or film is a non-perforated continuous microfiber web with microscopic pores large enough for moisture vapor to pass through, but small enough to resist air and liquid water. Microperforated membranes depend on mechanical pin-perforations and/or film laminations to build in properties. While both of the abovementioned types of sheet or film are permeable to water vapor, a sheet or film of the microporous type is presently preferred, as this type is less permeable to the passage of water or moisture in liquid or bulk form.

Useful vapor permeable, air barrier membrane will generally be a sheet or film, typically having a width (cross-direction or XD) in the range of about 30 to 250 cm, more typically about 60 to 160 cm; and a length (machine direction or MD) of about 5 to 80 m, more typically about 15 to 40 m, and is preferably provided in roll form.

In some advantageous exemplary embodiments, the membranes are self-adhering, comprising a vapor permeable, spun-bond, non-woven polyolefin fabric substrate coated (or more correctly partially coated) on one side (i.e., on one major surface or face) with an adhesive material, preferably a pressure sensitive adhesive material, more preferably a solventless or hot melt pressure sensitive adhesive. A removable release sheet or liner may advantageously overlay and contact the adhesive in order to prevent the adhesive from adhering to the back side (i.e., non-adhesive coated) major surface of the substrate in roll form, thereby preventing "blocking" of the rolled self-adhesive membrane. The release liner is removed prior to applying the membrane to an architectural structure. Alternatively, the back side major surface of the substrate may include an overlaid or overcoated low surface energy release layer or low adhesion backsize (LAB); such embodiments are preferably used in a linerless article.

### Exemplary Substrates

The utility of the present disclosure is relatively indifferent to the nature of the substrate. In some convenient embodiments, the substrate will be a web of indefinite length material, conveyed by conventional web handling techniques. Porous and non-porous polymeric materials can be employed, including solid films, woven, and non-woven webs, paper, and fabric. The substrate may include flexible glass sheets or webs. A discussion of how flexible glass sheets or webs may be successfully handled in these sorts of embodiments can be found in co-pending and co-assigned U.S. Patent Application No. 61/593,076, titled "Composite Glass Laminate and Web Processing Apparatus," (attorney docket number 69517US002), which is incorporated herein by reference in its entirety.

Vapor permeable, spun-bond, non-woven polyolefin substrate sheets are well-known and commercially available. They are typically made of polyethylene and/or polypropylene. The process of making a spun bond, non-woven polyolefin substrate sheet vapor permeable is also well known. Mukhopadhyay (Journal of Industrial Textiles 2008:37:225) provides a comprehensive review of waterproof breathable fabrics and their use.

In some exemplary embodiments, the self-adhering, vapor permeable air barrier membrane preferably meets air barrier requirements as described in ASTM E2179. The substrate sheets described in the present disclosure generally provide both water and air resistance barriers as defined by AC 38 (ICC-ES) and ASTM E 2179. In certain exemplary embodiments, the vapor permeance is preferably greater than 10 perms, more preferably greater than 15 perms, and most preferably greater than 20 perms (ASTM E96A at 75°F or about 24°C). It is generally a straightforward matter to select or fabricate a substrate sheet that meets the aforementioned criteria for air and water resistance, as well as vapor permeability.

In certain exemplary embodiments, the substrate is selected to be a microporous sheet or film. Suitable microporous sheets or films are preferably spunbonded or fibrous bonded polyolefin as described in U.S. Pat. Nos. 3,532,589 and 5,972,147. Preferred polyolefins are polyethylene and polypropylene. One suitable microporous sheet is commercially available under the trademark TYVEK™ from (available from E.I. DuPont deNemours Corp., Wilmington, DE). Other suitable microporous sheets include oriented polymeric films as described in U.S. Pat. No. 5,317,035, and which comprise ethylene-propylene block copolymers. Such films are commercially available as APTRA™ films (available from BP-Amoco Corp., Atlanta, Georgia).

The sheets or films may be reinforced with various types of scrim materials or may be laminated to other vapor permeable sheets or films, such as non-woven polypropylene or non-woven polyester for the purpose of improving strength and other physical properties. In general, the self-adhering air barrier membrane will typically have a thickness of 0.001 to 0.04 inches (about 25.4-1016 micrometers), preferably 0.001 to 0.025 inches (25.4 - 635 micrometers).

In additional alternative exemplary embodiments, the substrate is selected to be a (co)polymeric sheet or film. Suitable (co)polymeric materials include, for example, polyesters such as polyethylene terephthalate (PET), polylactic acid (PLA) and polyethylene naphthalate (PEN); polyimides such as KAPTON™ (available from E.I. DuPont deNemours Corp., Wilmington, DE); polycarbonates such as LEXAN (available from SABIC Innovative Plastics, Pittsfield, MA); cyclo olefin polymers such as ZEONEX or ZEONOR (available from Zeon Chemicals LP, Louisville, KY); and the like.

### Exemplary Coating Materials

The utility of the present disclosure is relatively indifferent to the nature of the coating materials, provided that their viscosity allows them to be impelled from the cavity through the needle tubes to the dispensing outlets. Adhesives, low-adhesion backsizings, surface modifying agents, and barrier layers are among the coating materials that may advantageously applied by way of the disclosed method.

Some useful coating materials include monomers or oligomers which are intended to be cured after being coated on the substrate. Such materials include those capable of being conveniently cured by applying heat, actinic radiation, ionizing radiation, or a combination thereof. Any form of electromagnetic radiation may be used, for example, the liquid compositions may be cured using UV-radiation and/or heat. Electron beam radiation may also be used. The liquid compositions described above are said to be cured using actinic radiation, i.e., radiation that leads to the production of photochemical activity. For example, actinic radiation may comprise radiation of from about 250 to about 700 nm. Sources of actinic radiation include tungsten halogen lamps, xenon and mercury arc lamps, incandescent lamps, germicidal lamps, fluorescent lamps, lasers and light emitting diodes. UV-radiation can be supplied using a high intensity continuously emitting system such as those available from Fusion UV Systems.

When curing with UV radiation, photoinitiators may be used in the coating materials. Photoinitiators for free radical curing include organic peroxides, azo compounds, quinines, nitro compounds, acyl halides, hydrazones, mercapto compounds, pyrylium compounds, imidazoles, chlorotriazines, benzoin, benzoin alkyl ethers, ketones, phenones, and the like. For example, the adhesive compositions may comprise ethyl-2,4,6-trimethylbenzoylphenylphosphinate available as LUCIRIN TPOL from BASF Corp. or 1-hydroxycyclohexyl phenyl ketone available as IRGACURE 184 from Ciba Specialty Chemicals. The photoinitiator is often used at a concentration of about 0.1 to 10 weight percent or 0.1 to 5 weight percent based on the weight of oligomeric and monomer material in the polymerizable composition.

The coating materials can optionally include one or more additives such as chain transfer agents, antioxidants, stabilizers, fire retardants, viscosity modifying agents, antifoaming agents, antistatic agents and wetting agents. If color is required for the optical adhesive, colorants such as dyes and pigments, fluorescent dyes and pigments, phosphorescent dyes and pigments can be used.

### Adhesives

In certain exemplary embodiments, the coating material is selected to be an adhesive material, more preferably a pressure sensitive adhesive (PSA) material, even more preferably a solventless or hot melt coatable PSA. Preferably, the substrate sheet is coated or partially coated on one side with a pressure sensitive adhesive. Any pressure sensitive adhesive used to adhere membranes to architectural structures (e.g., buildings) may be used. These include both vapor permeable and vapor impermeable pressure sensitive adhesives. An example of the latter is a rubber modified asphalt (bitumen) pressure sensitive adhesive or a synthetic rubber pressure sensitive adhesive. Such pressure sensitive adhesives are well known in the art.

Preferably, the adhesive is selected to be a solventless or hot melt adhesive; however, in some exemplary embodiments, solvent based adhesives, water based adhesives, or other types of adhesives, such as, for example, radiation-cured, e.g., ultraviolet (UV) radiation or electron-beam cured (co)polymers resulting from polymerizable monomers or oligomers) may be used. The applied adhesive is preferably tacky (i.e. sticky) and pressure sensitive.

Suitable hot melt adhesives may contain such ingredients as (co)polymers such as butyl rubber, styrene-butadiene-styrene (SBS), styrene-isoprene-styrene (SIS), styrene butadiene (SB), styrene-ethylene-butadiene-styrene (SEBS) and ethylenevinylacetate (EVA); resins such as those of the hydrocarbon and rosin types, natural and petroleum waxes, oils, bitumen and others.

Solvent-based adhesives may contain ingredients such as those listed above, dissolved or dispersed in a solvent vehicle.

Water based adhesives would normally be based on emulsions of (co)polymeric materials.

Suitable (co)polymeric materials include vinyl acetate and (meth)acrylic homopolymers and copolymers such as vinyl acetate acrylic, ethylene vinyl acetate as well as styrene acrylic, vinyl chloride acrylic, vinyl versatate and others.

From a production standpoint, the preferred adhesives are of the hot melt type which are simply melted for application and need not emit solvent which is an environmental pollutant and may require re-condensation.

Water based adhesives may have the disadvantage that they generally require the additional use of drying ovens or heat lamps to evaporate the water.

If a vapor permeable pressure sensitive adhesive is used, the substrate sheet may be completely coated on one side. If a vapor impermeable pressure sensitive adhesive is used, then the substrate sheet may be only partially coated with adhesive, typically in the range of about 20-85%, more typically about 30-80%, most typically 40-70%, of the surface area of the sheet. In other words, at least 15-80%, preferably 20-70%, most preferably 30-60%, of the surface area of the substrate sheet should be adhesive-free in order to maintain sufficient vapor permeability of the membrane.

The adhesive may suitably be applied at a thickness of 0.001 inches to 0.1 inch (about 2.54-254 mm), but is preferably applied at a thickness of 0.003 inches to 0.025 inches (about 7.62-63.5 mm) and most preferably at a thickness of 0.005 inches to 0.02 inches (about 12.7-50.8 mm).

As noted above, the adhesive may be protected with a strippable release sheet or liner to enable packaging in rolls. Suitable release sheets are paper or (co)polymer film sheets with an overlaying, low surface energy (e.g., silicone) release surface coating.

### Adhesive Patterns

To retain a desired level of water vapor permeance in the adhesive coated membrane, the adhesive is preferably applied to the vapor permeable membrane in a non-continuous film in order to leave parts, or spots or zones of the substrate surface uncoated with adhesive. In general, the adhesive film forms an adhesive sea on the membrane surface, with a multitude of membrane islands, surrounded by but not covered by the adhesive sea.

In order to prevent the lateral movement of air between the membrane and the substrate to which it is bonded, and through lap joints of the membrane, the adhesive coated areas of the membrane can be made to intersect to isolate the uncoated areas, thereby eliminating channels through which air can laterally move. This can be achieved by any number of patterns, such as intersecting circles with adhesive free centers, intersecting squares or rectangles of adhesive, intersecting strips in a checkered pattern, etc.

The adhesive may suitably be applied so as to cover 5% to 99% of the area of one side of the membrane, but is preferably applied to cover between 25% and 90% of the area, and most preferably between 50% and 80% of the area, to obtain the optimum balance of adhesion and vapor permeance for the sheet.

Partial coatings of adhesive may be applied in a random fashion or in a specific pattern. Some exemplary partial coatings of adhesive are described, for example, in U.S. Pat. Nos. 3,039,893, 3,426,754, 5,374,477, 5,593,771, 5,895,301, 6,495,229, and 6,901,712.

The operation of exemplary embodiments of the present disclosure will be further described with regard to the following non-limiting detailed Examples. These examples are offered to further illustrate the various specific and preferred embodiments and techniques. It should be understood, however, that many variations and modifications may be made while remaining within the scope of the present disclosure.

### EXAMPLES

These Examples are merely for illustrative purposes and are not meant to be overly limiting on the scope of the appended claims. Notwithstanding that the numerical ranges and parameters setting forth the broad scope of the present disclosure are approximations, the numerical values set forth in the specific examples are reported as precisely as possible. Any numerical value, however, inherently contains certain errors necessarily resulting from the standard deviation found in their respective testing measurements. At the very least, and not as an attempt to limit the application of the doctrine of equivalents to the scope of the claims, each numerical parameter should at least be construed in light of the number of reported significant digits and by applying ordinary rounding techniques.

### Summary of Materials

All parts, percentages, ratios, and the like in the Examples and the rest of the specification are by weight, unless noted otherwise. Solvents and other reagents used may be obtained from Sigma-Aldrich Chemical Company (Milwaukee, WI) unless otherwise noted.

### Example 1:

An apparatus generally as depicted in FIG. 1 was prepared. The dispensing outlets of the needle tubes were positioned adjacent to a conventional web handling line threaded up with a polyurethane (Estane, available from The Lubrizol Corporation of Wickliffe, OH) coated polyester nonwoven web (Reemay from Fiberweb Inc. of Old Hickory, TN) of indefinite length. The web was conveyed past the dispensing openings in the direction of the indefinite length at a line speed of 9 feet/minute (2.74 m/min).

A first coating material was prepared using 99 parts isooctyl acrylate (IOA), 1 part acrylic acid (AA) and 0.04 parts 2,2-dimethoxy-2-phenylacetophenone photoinitiator (Irgacure 651, available from BASF). This mixture was partially polymerized under a nitrogen atmosphere by exposure to ultraviolet radiation to provide a coatable syrup having a viscosity of about 4000 cps. An additional 0.26 parts of Irgacure 651 photoinitiator, 0.13 parts of 2,6-bis-trichoromethyl-6-(3,4-dimethoxyphenyl)-s-triazine and 6 parts Foral 85LB tackifier (a glycerol ester of highly hydrogenated wood rosin available from Pinova Inc.) were added to the syrup and mixed until all of the components had completely dissolved.

The first coating material was dispensed from the dispensing outlets onto the moving web while the needle tubes were oscillated at a rate of 6.7 Hz at a peak-to-peak amplitude of 25 mm. The pressure in the cavity of the distribution manifold was controlled to deliver the coating material at a coat weight of 32 grains/4"x6" (0.013 g/cm²). The first coating material was then exposed to ultraviolet radiation having a spectral output from 300-400 nm with a maximum at 351 nm in a nitrogen-rich environment. An intensity of about 9.0 mW/cm² was used during the exposure time, resulting in a total energy of 1800 mJ/cm². A pattern of pressure sensitive adhesive laid down in parallel sinusoids aligned in the longitudinal direction of the web was thus created.

### Example 2:

The set-up for this Example is generally similar to that of Example 1, except that a coating apparatus generally as depicted in FIG. 2 was employed. The coating material described in Example 1 was provided to both distribution manifolds, and the first dispensing outlets were reciprocated at a rate of 2.5 Hz, while the second dispensing outlets were kept stationary. A pattern generally as depicted in FIG. 4 was laid down on the substrate.

### Example 3:

The set-up for this Example is generally similar to that of Example 2, except that the first dispensing outlets were reciprocated at a rate of 2.5 Hz with an amplitude of 25 mm, while the second dispensing outlets were also reciprocated at a rate of 2.5 Hz with an amplitude of 12.5 mm. A pattern generally as depicted in FIG. 5 was laid down on the substrate.

Reference throughout this specification to "one embodiment," "certain embodiments," "one or more embodiments" or "an embodiment," whether or not including the term "exemplary" preceding the term "embodiment," means that a particular feature, structure, material, or characteristic described in connection with the embodiment is included in at least one embodiment of the certain exemplary embodiments of the present disclosure. Thus the appearances of the phrases such as "in one or more embodiments," "in certain embodiments," "in one embodiment" or "in an embodiment" in various places throughout this specification are not necessarily referring to the same embodiment of the certain exemplary embodiments of the present disclosure. Furthermore, the particular features, structures, materials, or characteristics may be combined in any suitable manner in one or more embodiments.

While the specification has described in detail certain exemplary embodiments, it will be appreciated that those skilled in the art, upon attaining an understanding of the foregoing, may readily conceive of alterations to, variations of, and equivalents to these embodiments. Accordingly, it should be understood that this disclosure is not to be unduly limited to the illustrative embodiments set forth hereinabove. In particular, as used herein, the recitation of numerical ranges by endpoints is intended to include all numbers subsumed within that range (e.g., 1 to 5 includes 1, 1.5, 2, 2.75, 3, 3.80, 4, and 5). In addition, all numbers used herein are assumed to be modified by the term "about."

## Claims

1. A method of applying a coating material to a substrate (60), comprising:
providing a distribution manifold (22) having a cavity (24) and a plurality of dispensing outlets (30) in fluid communication with the cavity (24);
creating relative motion between a substrate and the dispensing outlets (30) in a first direction (D); and
dispensing coating material from the dispensing outlets (30) while simultaneously translating the plurality of dispensing outlets (30) in a second direction (C) non-parallel to the first direction,
wherein the distribution manifold is translated in the second direction, thereby translating the plurality of dispensing outlets in the second direction,
**characterized in that** the plurality of dispensing outlets (30) is comprised of a plurality of needle tubes (26) in fluid communication with the cavity (24), wherein the distribution manifold (22) further comprises an alignment bar (32) for securing a relative spacing between each needle tube (26), and further wherein the relative spacing is substantially uniform.

2. The method according to claim 1 wherein the substrate (60) is optionally moved in the first direction (D) relative to the distribution manifold (22).

3. The method according to claim 1 or 2 wherein the plurality of dispensing outlets (30) forms an array of dispensing outlets.

4. The method according to any one of the preceding claims wherein the second direction (C) is perpendicular to the first direction (D).

5. The method according to any one of the preceding claims wherein translating is in an oscillatory fashion.

6. The method according to any one of the preceding claims further comprising providing a second distribution manifold (20a) having a cavity and a plurality of second dispensing outlets (30a) in fluid communication with the cavity, and dispensing a second coating material from the second dispensing outlets.

7. The method according to claim 6 wherein the second distribution manifold (20b) is simultaneously translated in a direction non-parallel to the first direction (D).

8. The method according to claim 6 or 7 wherein coating-free zones completely surrounded by the first and the second coating materials are formed on the substrate (60).

9. The method according to claim 8 wherein the first and second coating materials are different.

10. The method according to claim 1 wherein the translating is accomplished by moving the alignment bar (32).

## Patentansprüche

1. Verfahren zum Aufbringen eines Beschichtungsmaterials auf ein Substrat (60), umfassend:
Bereitstellen eines Verteilers (22) mit einem Hohlraum (24) und einer Mehrzahl von Abgabeauslässen (30) in Fluidverbindung mit dem Hohlraum (24);
wodurch eine relative Bewegung zwischen einem Substrat und den Abgabeauslässen (30) in eine erste Richtung (D) entsteht; und
Abgeben von Beschichtungsmaterial aus den Abgabeauslässen (30) unter gleichzeitigem Verschieben der Mehrzahl von Abgabeauslässen (30) in eine zweite Richtung (C), die nicht parallel zu der ersten Richtung ist,
wobei der Verteiler in die zweite Richtung verschoben wird, wodurch die Mehrzahl von Abgabeauslässen in die zweite Richtung verschoben wird,
**dadurch gekennzeichnet, dass** die Mehrzahl von Abgabeauslässen (30) aus einer Mehrzahl von Nadelrohren (26) in Fluidverbindung mit dem Hohlraum (24) besteht, wobei der Verteiler (22) ferner einen Ausrichtungsstab (32) zur Sicherung eines relativen Abstands zwischen jedem Nadelrohr (26) umfasst und wobei ferner der relative Abstand im Wesentlichen gleich ist.

2. Verfahren nach Anspruch 1, wobei das Substrat (60) wahlweise in die erste Richtung (D) relativ zu dem Verteiler (22) bewegt wird.

3. Verfahren nach Anspruch 1 oder 2, wobei die Mehrzahl von Abgabeauslässen (30) eine Anordnung von Abgabeauslässen bildet.

4. Verfahren nach einem der vorstehenden Ansprüche, wobei die zweite Richtung (C) senkrecht zu der ersten Richtung (D) ist.

5. Verfahren nach einem der vorstehenden Ansprüche, wobei das Verschieben auf oszillierende Weise erfolgt.

6. Verfahren nach einem der vorstehenden Ansprüche, ferner umfassend das Bereitstellen eines zweiten Verteilers (20a) mit einem Hohlraum und einer Mehrzahl von zweiten Abgabeauslässen (30a) in Fluidverbindung mit dem Hohlraum und das Abgeben eines zweiten Beschichtungsmaterials aus den zweiten Abgabeauslässen.

7. Verfahren nach Anspruch 6, wobei der zweite Verteiler (20b) gleichzeitig in eine Richtung verschoben wird, die nicht parallel zu der ersten Richtung (D) ist.

8. Verfahren nach Anspruch 6 oder 7, wobei auf dem Substrat (60) vollständig von dem ersten und dem zweiten Beschichtungsmaterial umgebene, beschichtungsfreie Zonen gebildet werden.

9. Verfahren nach Anspruch 8, wobei das erste und das zweite Beschichtungsmaterial verschieden sind.

10. Verfahren nach Anspruch 1, wobei das Verschieben durch Bewegen des Ausrichtungsstabs (32) ausgeführt wird.

## Revendications

1. Procédé d'application d'un matériau de revêtement sur un substrat (60), comprenant :
la fourniture d'un collecteur de distribution (22) ayant une cavité (24) et une pluralité de sorties de distribution (30) en communication fluidique avec la cavité (24) ;
la création d'un mouvement relatif entre un substrat et les sorties de distribution (30) dans une première direction (D) ; et
la distribution de matériau de revêtement à partir des sorties de distribution (30) tout en translatant simultanément la pluralité de sorties de distribution (30) dans une deuxième direction (C) non parallèle à la première direction,
dans lequel le collecteur de distribution est translaté dans la deuxième direction, translatant de ce fait la pluralité de sorties de distribution dans la deuxième direction,
**caractérisé en ce que** la pluralité de sorties de distribution (30) est constituée d'une pluralité de tubes d'aiguilles (26) en communication fluidique avec la cavité (24), dans lequel le collecteur de distribution (22) comprend en outre une barre d'alignement (32) pour assurer un espacement relatif entre chaque tube d'aiguille (26), et en outre dans lequel l'espacement relatif est sensiblement uniforme.

2. Procédé selon la revendication 1 dans lequel le substrat (60) est éventuellement déplacé dans la première direction (D) par rapport au collecteur de distribution (22).

3. Procédé selon la revendication 1 ou 2 dans lequel la pluralité de sorties de distribution (30) forme un réseau de sorties de distribution.

4. Procédé selon l'une quelconque des revendications précédentes dans lequel la deuxième direction (C) est perpendiculaire à la première direction (D).

5. Procédé selon l'une quelconque des revendications précédentes dans lequel la translation est de façon oscillatoire.

6. Procédé selon l'une quelconque des revendications précédentes comprenant en outre la fourniture d'un deuxième collecteur de distribution (20a) ayant une cavité et une pluralité de deuxièmes sorties de distribution (30a) en communication fluidique avec la cavité, et la distribution d'un deuxième matériau de revêtement à partir des deuxièmes sorties de distribution.

7. Procédé selon la revendication 6 dans lequel le deuxième collecteur de distribution (20b) est translaté simultanément dans une direction non parallèle à la première direction (D).

8. Procédé selon la revendication 6 ou 7 dans lequel les zones exemptes de revêtement entourées complètement par les premier et deuxième matériaux de revêtement sont formées sur le substrat (60).

9. Procédé selon la revendication 8 dans lequel les premier et deuxième matériaux de revêtement sont différents.

10. Procédé selon la revendication 1 dans lequel la translation est accomplie en déplaçant la barre d'alignement (32).
